# EUROPEAN PATENT SPECIFICATION

(11) **EP 2 568 290 B1**
(45) Date of publication and mention of the grant of the patent: **22.02.2017**
(21) Application number: 11382290.2
(22) Date of filing: 12.09.2011
(51) Int. Cl.: G01N 33/574

(54) **Methods for prognosis of diffuse large B-cell lymphoma**
Verfahren zur Vorhersage diffuser großer B-Zellen-Lymphome
Procédés de pronostic de lymphome de lymphocytes B à large diffusion

(43) Date of publication of application: 13.03.2013
(73) Proprietor: Atrys Health, SA, 28001 Madrid (ES); Fundació Clínic per a la Recerca Biomèdica, 08036 Barcelona (ES)
(72) Inventor: Donovan, Michael J., E-08025 Barcelona (ES); Erill Sagalés, Nadina, E-08025 Barcelona (ES); Puig, Pere, E-08025 Barcelona (ES); Colomer Valero, M Anna, E-08025 Barcelona (ES); Campo, Elías, E-08036 Barcelona (ES); Colomo, Luis, E-08036 Barcelona (ES)
(74) Representative: ABG Patentes, S.L.

(56) References cited:
- WO-A1-2009/149297
- US-A1- 2008 260 641
- LARS M. PEDERSEN ET AL: "Serum levels of inflammatory cytokines at diagnosis correlate to the bcl-6 and CD10 defined germinal centre (GC) phenotype and bcl-2 expression in patients with diffuse large B-cell lymphoma", BRITISH JOURNAL OF HAEMATOLOGY, vol. 128, no. 6, 1 March 2005 (2005-03-01) , pages 813-819, XP055018177, ISSN: 0007-1048, DOI: 10.1111/j.1365-2141.2005.05393.x
- DOU H -J ET AL: "Clinical significances of serum levels of VEGF and the relationship with IPI in the patients with diffuse large B-cell lymphoma", JOURNAL OF LEUKEMIA AND LYMPHOMA 20090325 EDITORIAL BOARD OF JOURNAL OF LEUKEMIA AND LYMPHOMA CHN LNKD- DOI:10.3760/CMA.J.ISSN.1009-9921.2009.03.0 10, vol. 18, no. 3, 25 March 2009 (2009-03-25) , pages 155-157, XP009156104, ISSN: 1009-9921
- JI D -M ET AL: "The relationships between the serum levels of [beta]2-MG, VEGF, bFGF, and IL-6 in patients with diffuse large B cell lymphoma and parameters of International Prognostic Index", TUMOR 20070525 CN, vol. 27, no. 5, 25 May 2007 (2007-05-25), pages 402-405, XP009156048, ISSN: 1000-7431
- PAYDAS S ET AL: "Prognostic significance of EBV-LMP1 and VEGF-A expressions in non-Hodgkin's lymphomas", LEUKEMIA RESEARCH, NEW YORK,NY, US, vol. 32, no. 9, 1 September 2008 (2008-09-01), pages 1424-1430, XP022659309, ISSN: 0145-2126, DOI: 10.1016/J.LEUKRES.2008.01.008 [retrieved on 2008-02-20]
- RIIHIJARVI SARI ET AL: "High Serum Vascular Endothelial Growth Factor (VEGF) Level Is An Adverse Prognostic Factor In High Risk Diffuse Large B-Cell Lymphoma (DLBCL) Patients Treated with Dose-Dense Chemoimmunotherapy and Systemic CNS Prophylaxis. Results From a Nordic Phase II Study", BLOOD, vol. 116, no. 21, November 2010 (2010-11), page 1686, XP009156098, & 52ND ANNUAL MEETING OF THE AMERICAN-SOCIETY-OF-HEMATOLOGY (ASH); ORLANDO, FL, USA; DECEMBER 04 -07, 2010
- TANG QIONG-LAN ET AL: "[Expression of VEGF and its significance in primary diffuse large B-cell lymphoma of the female genital system].", SICHUAN DA XUE XUE BAO. YI XUE BAN = JOURNAL OF SICHUAN UNIVERSITY. MEDICAL SCIENCE EDITION JAN 2009 LNKD- PUBMED:19292043, vol. 40, no. 1, January 2009 (2009-01), pages 48-51, XP009156092, ISSN: 1672-173X
- LOSSOS IZIDORE S ET AL: "Prognostic biomarkers in diffuse large B-cell lymphoma", JOURNAL OF CLINICAL ONCOLOGY,, vol. 24, no. 6, 20 February 2006 (2006-02-20), pages 995-1007, XP009156004, ISSN: 1527-7755, DOI: 10.1200/JCO.2005.02.4786 [retrieved on 2006-01-17]
- J. N. WINTER: "Prognostic significance of Bcl-6 protein expression in DLBCL treated with CHOP or R-CHOP: a prospective correlative study", BLOOD, vol. 107, no. 11, 1 June 2006 (2006-06-01) , pages 4207-4213, XP055029469, ISSN: 0006-4971, DOI: 10.1182/blood-2005-10-4222
- I. S. LOSSOS: "Expression of a single gene, BCL-6, strongly predicts survival in patients with diffuse large B-cell lymphoma", BLOOD, vol. 98, no. 4, 15 August 2001 (2001-08-15), pages 945-951, XP055029462, ISSN: 0006-4971, DOI: 10.1182/blood.V98.4.945
- HANS C P ET AL: "Confirmation of the molecular classification of diffuse large B-cell lymphoma by immunohistochemistry using a tissue microarray", BLOOD, AMERICAN SOCIETY OF HEMATOLOGY, US, vol. 103, no. 1, 22 September 2003 (2003-09-22), pages 275-282, XP002322639, ISSN: 0006-4971, DOI: 10.1182/BLOOD-2003-05-1545
- J IQBAL ET AL: "Distinctive patterns of BCL6 molecular alterations and their functional consequences in different subgroups of diffuse large B-cell lymphoma", LEUKEMIA, vol. 21, no. 11, 1 November 2007 (2007-11-01), pages 2332-2343, XP055030096, ISSN: 0887-6924, DOI: 10.1038/sj.leu.2404856
- SVERKER HASSELBLOM ET AL: "Expression of CD68 tumor-associated macrophages in patients with diffuse large B-cell lymphoma and its relation to prognosis", PATHOLOGY INTERNATIONAL, vol. 58, no. 8, 1 August 2008 (2008-08-01) , pages 529-532, XP055029516, ISSN: 1320-5463, DOI: 10.1111/j.1440-1827.2008.02268.x
- P. N. MEYER ET AL: "The Stromal Cell Marker SPARC Predicts for Survival in Patients With Diffuse Large B-Cell Lymphoma Treated With Rituximab", AMERICAN JOURNAL OF CLINICAL PATHOLOGY, vol. 135, no. 1, 20 December 2010 (2010-12-20), pages 54-61, XP055029500, ISSN: 0002-9173, DOI: 10.1309/AJCPJX4BJV9NLQHY
- QI-CHUN CAI ET AL: "High expression of tumor-infiltrating macrophages correlates with poor prognosis in patients with diffuse large B-cell lymphoma", MEDICAL ONCOLOGY, 24 December 2011 (2011-12-24), XP055029917, ISSN: 1357-0560, DOI: 10.1007/s12032-011-0123-6
- N. A. JOHNSON ET AL: "Diffuse large B-cell lymphoma: reduced CD20 expression is associated with an inferior survival", BLOOD, vol. 113, no. 16, 16 April 2009 (2009-04-16), pages 3773-3780, XP055030111, ISSN: 0006-4971, DOI: 10.1182/blood-2008-09-177469
- GEUNDOO JANG ET AL: "Addition of rituximab to the CHOP regimen has no benefit in patients with primary extranodal diffuse large B-cell lymphoma", THE KOREAN JOURNAL OF HEMATOLOGY, vol. 46, no. 2, 1 January 2011 (2011-01-01), page 103, XP055029527, ISSN: 1738-7949, DOI: 10.5045/kjh.2011.46.2.103

## Description

### FIELD OF THE INVENTION

The invention belongs to the field of methods for the prognosis of a patient suffering from diffuse large B-cell lymphoma in response to a therapy. The invention also discloses predicting the clinical response of a patient suffering from diffuse large B-cell lymphoma in response to a therapy.

### BACKGROUND OF THE INVENTION

Diffuse large B-cell lymphoma (DLBCL) is one of the more common (about 40%) and aggressive subtypes of non-Hodgkin's lymphoma (median survival of 1 - 2 years if untreated), characterized by a marked degree of morphologic and clinical heterogeneity. The pathogenesis of DLBCL is both complex and heterogeneous, and pathogenetic mechanisms remain largely unknown. Although many DLBCL patients respond to anthracycline-based chemotherapy and even the monoclonal antibody rituximab (about 80%), approximately half of all patients eventually die of their disease. Several clinical factors have prognostic significance including age, stage, systemic symptoms, performance status, tumor burden and serum lactate dehydrogenase (LDH) levels. These features make up the international prognostic index (IPI) which is the 'gold-standard' for predicting response to therapy and overall survival. This observed variation in response to chemotherapy across multiple patient groups, even with inclusion of the IPI, has suggested that DLBCL may actually represent multiple disease forms with differing outcomes based on response to treatment.

The original observations from DNA microarray array technology (Alizadeh et al., 2000, Nature 403:503-11; Shipp et al., Nat Med 8:68-74) had identified two molecularly and clinically distinct forms of DLBCL with gene expression patterns indicative of different stages of B-cell differentiation and survival outcomes. The disease heterogeneity hypothesis was further elucidated by Rosenwald et al. (N Engl J Med, 2002, 346:1937-47) with the identification of two distinct sub-groups with expression profiles representing either germinal-center B-cell type or activated B-cells. With a 'lymphochip' DNA microarray, four gene signature subgroups were identified in a cohort of patients diagnosed with DLBCL and treated with anthracycline-based chemotherapy. These broad expression classes (e.g., Germinal center B-cell type, MHC class II, lymph node and proliferation) segregated patient groups with respect to overall survival, and reflected biologic states associated with cell growth and the immune response. Subsequently, Rimsza et al. (Blood, 2008, 112:3425-33), observed that low RNA expression levels of HLA-DR and high levels of MYC were associated with a shortened survival time, also in agreement with previous proposed mechanisms of tumor cell proliferation, and host response.

The results from several recently completed clinical trial and laboratory studies have implicated a number of signal transduction pathways in the malignant growth of DLBCL including PI3K, BCR and PKC. Notable has been the association of PKC beta over-expression either by RNA or protein and adverse prognosis in patients with DLBCL. These studies have recently been confirmed using an R-CHOP treated patient group where PKC-beta II mRNA levels have independent prognostic significance in DLBCL patients treated with immunochemotherapy (Riihijarvi S. et al., Mod. Pathol., 2010, 23:686-93). In sum, the efficacious treatment of patients with either *de novo* or refractory DLBCL requires a more complete understanding of the assorted mechanisms and pathways which drive the disease process.

The incorporation of rituximab combined with CHOP (cyclophosphamide, doxorubicin, vincristine, and prednisone), known as R-CHOP or CHOP-R, as first-line treatment (for elderly patients) for DLBCL has altered the natural history of the disease although a significant number (30%) of patients still fail to respond to initial therapy or progress shortly after the completion of initial treatment. Recent developments in RNA expression profiling on formalin fixed, paraffin embedded DLBCL tumor samples (Rimsza et al., 2008, Blood, 112:3425-33; Mallumbres et al., 2008, Blood, 111:5509-14) from patients treated with CHOP or R-CHOP (rituximab and CHOP) have confirmed that previous identified predictive gene signatures were relevant with respect to current treatments. These studies also established that molecular techniques such as quantitative nuclease protection assays and real time-polymerase chain reaction (RTPCR) on formalin-fixed paraffin-embedded (FFPE) primary tumor samples were able to provide meaningful prognostic genomic information.

There are different approaches in the state of the art. WO 2010/121231 describes a method based on the determination of the expression levels of at least one marker gene selected from the following BCL6, IFITM1, CD40, RGS13, VNN2, LMO2, CD79B, CD22, BTG2, IGF1R, CD44, CTSC, EPDR1, UAP1, and PUS7, for predicting or assessing responsiveness of a patient having B-cell lymphoma (including DLBCL) to treatment with anti-CD40 antibodies.

WO 2009/149297 discloses a method for determining the expression levels of GCET1, HLA-DQA1, HLA-DRB, HLA-DRA, ACTN1, COL3A1, PLAU, MYC, BCL6, LMO2, PDCD4, and SOD2, for prognosing treatment outcome in DLBCL patients, diagnosing DLBCL and monitoring efficacy of a CHOP or R-CHOP treatment.

Gratzinger et al., (Br. J. Haematol. 2010 January; 148(2):235-244) provided immunohistochemistry (IHC) expression data of CD34, VEGF, VEGFR1 and VEGFR2 on a series of DLBCL tumor specimens from patients treated with CHOP. In this study, VEGF expression in lymphoma cells was not predictive of overall survival (OS) on univariate or multivariate analysis with VEGFR1, VEGFR2 and IPI. In addition, microvessel density with CD34 was also not found to be predictive. On the contrary, VEGFR1 and VEGFR2 along with the phosphorylated VEGFR2 form were found to be highly predictive of good and poor OS, respectively. Previous studies have linked high VEGF protein and high VEGFR1 in lymphoma cells as associated with good overall survival in patients who have received anthracycline-based therapy. The hypothesis is that this autocrine loop represents a survival or proliferation pathway which is quite susceptible to anthracyclines.

Lossos et al., (2006, J. Clin. Oncol. 24:995-1007) discloses an overview on prognostic biomarkers in DLBCL. BCL6 and VEGF are disclosed as prognostic biomarkers.

Winter et al., (2006, Blood 107:4207-13) discloses that BCL6 protein expression, as measured by immunostaining, is a powerful predictor of outcome in patients treated with CHOP chemotherapy. In contrast, BCL6 was not a prognostic marker among patients treated with R-CHOP. A commercial reagent for detection of BCL6 is also disclosed.

Lossos et al., (2001, Blood 98:945-51) discloses that high BCL6 mRNA expression or protein expression by immunohistochemistry is a new favorable prognostic factor in DLBCL and should be used in the stratification and the design of risk-adjusted therapies for patients with DLBCL.

Hans et al., (2003, Blood 103:275-82) discloses that BCL6 expression was associated with better overall survival and event free survival.

There continues to be a clinical need to use biologic markers to predict outcome and define therapies for newly diagnosed patients with DLBCL. There also remains a significant need for the development of more standardized methods which incorporate quantitative assays for understanding marker expression.

### BRIEF DESCRIPTION OF THE INVENTION

In a first aspect, the invention relates to an *in vitro* method for prognosis of a patient suffering from diffuse large B-cell lymphoma (DLBCL), which comprises determining in a sample from said patient the level of BCL6 protein by quantitative immunofluorescence, wherein said patient is under therapy with CHOP-R, and wherein a high level of BCL6 expression in said sample is indicative of a bad prognosis and a low level of BCL6 expression in said sample is indicative of a good prognosis.

It is also disclosed that:
- a high level of VEGF expression in said sample is indicative of a bad prognosis and a low level of VEGF expression in said sample is indicative of a good prognosis,
- a high level of CD68 expression or CD68⁺ cells in said sample is indicative of a bad prognosis and a low level of CD68 expression or CD68⁺ cells in said sample is indicative of a good prognosis, and/or
- a high level of CD20 expression or CD20⁺ cells in said sample is indicative of a good prognosis and a low level of CD20 expression or CD20⁺ cells in said sample is indicative of a bad prognosis.

In a second aspect, the document also discloses a method for selecting a patient suffering from DLBCL for individual therapy, comprising the method of the first aspect, wherein said patient is under a DLBCL therapy or not, and wherein a good prognosis selects said patient for said individual therapy and/or a bad prognosis rejects said patient for said individual therapy.

In a third aspect, the document also discloses a kit comprising reagents for analyzing a sample from a subject for determining the levels of one or more biomarkers selected from the group consisting of VEGF, BCL6, CD68, and CD20 according to the method of the first and/or the second aspect.

### BRIEF DESCRIPTION OF THE FIGURES

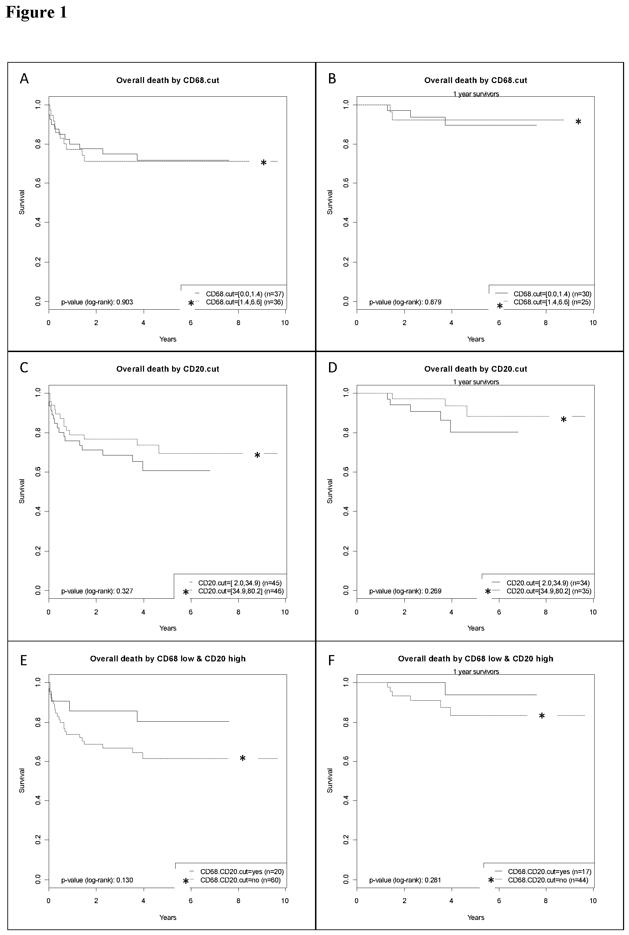

Figure 1: Kaplan-Meier survival curves for CD68 and CD20 predicting OS (A,C,E) and survival at one year (B,D,F), as independent and combined predictors. As illustrated, neither CD20 nor CD68 are statistically significant as independent predictors (B); however when combined with CD20, especially for predicting OS, there is trend towards significance.

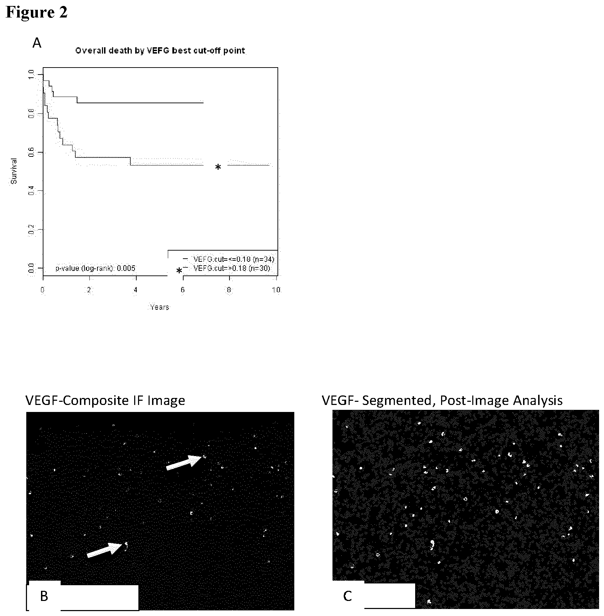

Figure 2: A. Overall survival was significantly reduced with increasing VEGF levels based on a VEGF cut-point of 0.18 (p=0.005). B. Immunofluorescent composite image of high VEGF expression [yellow (clear points), white arrows] in malignant lymphocytes and endothelial cells. C. Post-processed, segmented image of normalized VEGF (0.69, yellow), relative to total tumor area (DAPI, blue).

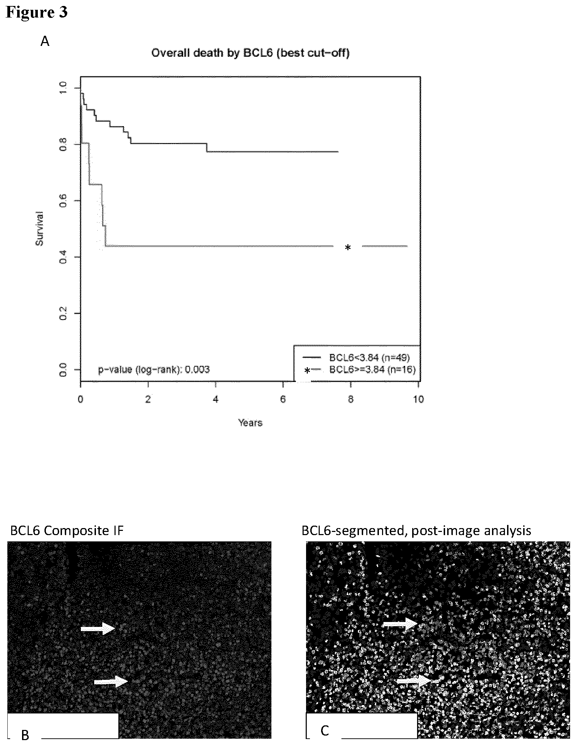

Figure 3: A. Overall survival (OS) is significantly reduced with increasing BCL6 based on a BCL6 cut-point (≤ 3.84, low risk; > 3.84, high risk) for OS (p=0.003). B. Immunofluorescent image of DAPI stained nuclei (blue) and BCL6 expression (magenta, white arrows) in malignant lymphocytes. C. Post-processed, segmented image representing normalized BCL6 (21.18) co-localized with DAPI relative to total tumor area (yellow, BCL6+DAPI; magenta, BCL6; blue, DAPI).

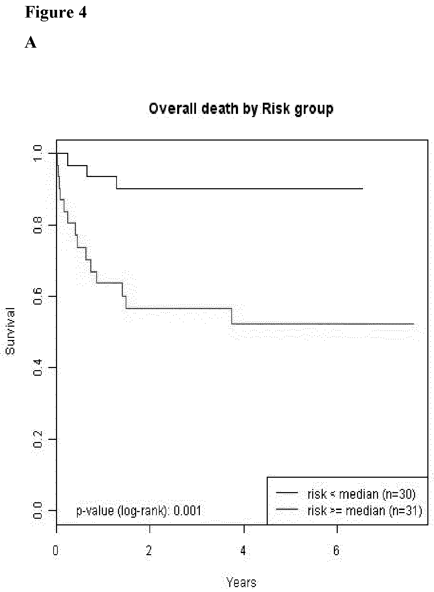

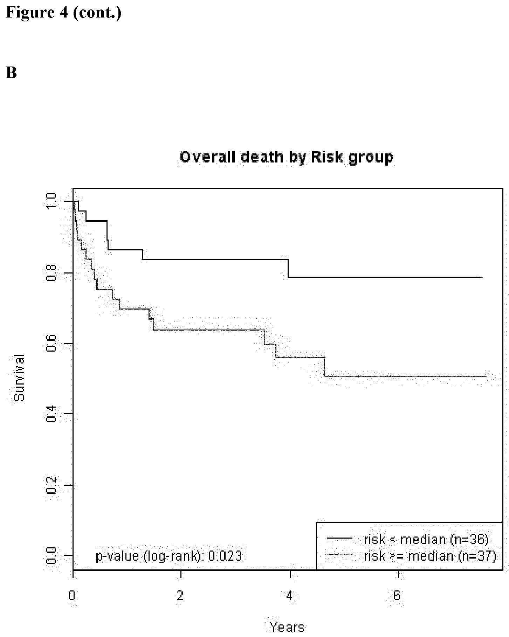

Figure 4: A. Kaplan-Meier survival curve representing a multivariate model which includes age, IPI status and VEGF levels. The stratification of patients was performed using the median of the computed risk from the Cox model to define two risk groups (black line represents the group with less estimated risk while the red line is the higher risk group). B. Kaplan-Meier curve on the same patient group as in Figure 4A, stratified by age and IPI status only. This illustrates the importance of including the quantitative biomarker VEGF in the stratification of patients with respect to OS.

### DETAILED DESCRIPTION OF THE INVENTION

In the present document, methods are provided for prognosis of a patient afflicted with DLBCL, comprising determining the levels of expression of specific biomarkers either alone, or in combinations thereof, and/or in combinations with clinical variables. In addition, methods are also disclosed for predicting response of DLBCL patients to DLBCL therapy, particularly CHOP-R therapy.

The methods of the invention are useful in the prognosis of patients with diffuse large B-cell lymphoma (DLBCL). The present invention may be carried out in different modes, which are outlined in the following.

### Definitions

The following definitions are set forth to illustrate and define the meaning and scope of various terms and expressions used to describe the invention therein.

The term "age", as used herein, refers to the length of time that one has existed or the duration of life.

The term "biomarker" refers to any biological entity, preferably mRNA, protein or cells, the occurrence or amount of which is characteristic for a specific situation, for example a disease such as DLBCL.

The term "BCL6", as used herein, refers to the human B-cell lymphoma 6 protein as shown in the UniProt database under accession number P41182 at date 4^{th} August 2011. In a preferred embodiment, the optimal cut-point value for BCL6 is 3.84, although generally a range of 0.10 to 16.3 (0.08 to 3.84, 90% confidence interval - the 90% confidence interval was used due to the asymmetric distribution of BCL6 cut-point) is preferred, alternatively, a range of 1 to 10 or 2.4 to 7 using the statistical method described in the examples (preferably Kaplan-Meier), p preferably ranging from 0.01 to 0.25, more preferably from 0.03 to 0.1 and most preferably being 0.05.

The term "CD20", as used herein, refers to the B-lymphocyte antigen Cluster of Differentiation 20 as shown in the UniProt database under accession number P11836. In a preferred disclosure, the optimal cut-point for CD20 is 34.9, although generally a range of 7.7 to 56.4 (95% confidence interval) is preferred, alternatively, a range of 18 to 42 or 30 to 40 using the statistical method described in the examples (preferably Kaplan-Meier), p preferably ranging from 0.01 to 0.25, more preferably from 0.03 to 0.1 and most preferably being 0.05.

The term "CD20+ cells", as used herein, refers to B cells expressing CD20 on their surface. "B cells" are lymphocytes that play a large role in the humoral immune response (as opposed to the cell-mediated immune response, which is governed by T cells). The principal functions of B cells are to make antibodies against antigens, perform the role of antigen-presenting cells (APCs) and eventually develop into memory B cells after activation by antigen interaction. B cells are an essential component of the adaptive immune system. CD20 is expressed on all stages of B cell development except the first and last; it is present from late pro-B cells through memory cells, but not on either early pro-B cells or plasma blasts and plasma cells.

The term "CD68", as used herein, refers to the Cluster of Differentiation 68 protein as shown in the UniProt database under accession number P34810 at date 4^{th} August 2011. In a preferred disclosure, the optimal cut-point for CD68 is 3.66, although generally a range of 0.18 to 3.66 (95% confidence interval) is preferred, alternatively, a range of 1 to 10 or 2.4 to 7 using the statistical method described in the examples (preferably Kaplan-Meier), p preferably ranging from 0.01 to 0.25, more preferably from 0.03 to 0.1 and most preferably being 0.05.

The term "CD68+ cells", as used herein, refers to tumor-infiltrating macrophages or tumor-associated macrophages. "macrophages" are white blood cells produced by the differentiation of monocytes in tissues. Monocytes and macrophages are phagocytes. Macrophages function in both non-specific defense (innate immunity) as well as help initiate specific defense mechanisms (adaptive immunity) of vertebrate animals. Their role is to phagocytose (engulf and then digest) cellular debris and pathogens, either as stationary or as mobile cells. They also stimulate lymphocytes and other immune cells to respond to pathogens. They are specialized phagocytic cells that attack foreign substances, infectious microbes and cancer cells through destruction and ingestion. Macrophages can be identified by specific expression of a number of proteins including CD14, CD11b, F4/80 (mice)/EMR1 (human), lysozyme M, MAC-1/MAC-3 and CD68 by conventional methods known by the person skilled in the art.

The term "clinical parameter" as used herein, refers to the demographic (illustrative, non-limitative, examples include age and gender) and clinical-pathologic features (illustrative, non-limitative, examples include clinical stage, serum LDH, and tumor burden) that defines the patient, generates the IPI score and/or reflects on the manifestation of their disease. Preferably, said clinical parameter(s) is/are selected from the group consisting of IPI, age, complete/partial response to therapy, rituximab treatment, gender, lack of recurrence in 1 year, nodal restricted disease, clinical stage (e.g. ECOG status), tumour burden, and serum LDH. More preferably said clinical parameter(s) is/are IPI and/or age.

The term "clinical stage", as used herein, refers to the extent of spread of the disease. The stage often takes into account the size of a tumor, how deeply it has penetrated, whether it has invaded adjacent organs, how many lymph nodes it has metastasized to (if any), and whether it has spread to distant organs. Staging of cancer is the most important predictor of survival, and cancer treatment is primarily determined by staging. Thus, staging does not change with progression of the disease as it is used to assess prognosis. Patients' cancer, however, may be restaged after treatment but the staging established at diagnosis is rarely changed. Clinical stage is based on all of the available information obtained before a surgery to remove the tumor. Thus, it may include information about the tumor obtained by physical examination, radiologic examination, and endoscopy. It may include additional information gained by examination of the tumor microscopically by a pathologist.

The term "cohort", as used herein, refers to a group of patients who share a common characteristic or experience within a defined period, i.e. they suffer from DLBCL. The cohort comprises at least 20, at least 30, at least 50, at least 75, at least 100 or at least 150 of such patients.

The term "correlating" or "correlation", as used herein, refers to the combination of a biomarker with another biomarker or a clinical parameter for making a prognosis. Thereby, the stringency for each biomarker/clinical parameter cut-off value is reduced.

The expression "determining the level of a biomarker" refers to determining the level of expression of a biomarker and/or the number of cells carrying a biomarker on its surface (i.e. a cell surface marker). Therein, the level of expression refers to the level of mRNA and/or the level of protein and/or the number of cells carrying a biomarker on its surface.

The term "diffuse large B-cell lymphoma (DLBCL)", as used herein, refers to a type of aggressive non-Hodgkin lymphoma, which accounts for approximately 40% of lymphomas among adults. It is typically diagnosed between the ages of 25 and 40 years. As with most non-Hodgkin lymphomas, there is a male predominance. Of all cancers involving the same class of blood cell, 31% of cases are DLBCL. Three major subtypes of DLBCL have been identified based on their genetic activity:
- activated (ABC-DLBCL), with a pattern of genetic expression that is similar to healthy, activated B cells,
- germinal center (GCB-DLBCL), with a pattern of genetic expression that is similar to germinal center B cells and a chromosomal translocation involving the gene BCL-2; this type has a relatively favorable prognosis, and
- primary mediastinal B-cell lymphoma (PMBL), where the cancer is found in the thymus gland and lymph nodes behind the sternum (mediastinum), the area between the lungs, in the middle of the chest.

The stage of non-Hodgkin lymphoma describes how many groups of lymph nodes are affected, where they are in the body and whether other organs such as the bone marrow or liver are involved:
- Stage 1: The lymphoma is only in one group of lymph nodes, in one particular area of the body.
- Stage 2: More than one group of lymph nodes is affected, but all the affected nodes are contained within either the upper or lower half of the body. The upper half of the body is above the diaphragm (the sheet of muscle underneath the lungs), and the lower half is below it.
- Stage 3: The lymphoma is in lymph nodes above and below the diaphragm. The spleen is considered a lymph node in this staging system.
- Stage 4: The lymphoma has spread beyond lymph nodes to other organs such as the bones, liver or lungs.

As well as giving each stage a number, either the letter A or B can be used to show whether or not specific symptoms are present. If there is weight loss, fevers or night sweats, the letter B will be added next to the stage; otherwise, the letter A is added. Sometimes the lymphoma can start in areas outside the lymph nodes. This is called extranodal lymphoma, and the stage will include the letter E (for extranodal).

The expression "disease-free survival" is well known to one of skill in the art and means living free of the disease being monitored; for example, if biomarkers expression is used to diagnose or monitor DLBCL, disease-free survival would mean free from detectable DLBCL.

The expression "event-free survival" is well known to one of skill in the art and means living without the occurrence of a particular group of defined events (e.g., progression of cancer) after a particular action (e.g., treatment).

The term "gender", as used herein, refers to the sexual identity or the condition of being female or male. Preferably, it is determined by genetic means and not by physical appearance.

The term "International Prognostic Index (IPI) score", as used herein, refers to the clinical tool developed by oncologists to aid in predicting the prognosis of patients with aggressive non-Hodgkin's lymphoma. The IPI score was developed through a retrospective analysis performed on 2031 patients with aggressive non-Hodgkin's lymphoma, of all ages, treated with a doxorubicin-based chemotherapy regimen such as CHOP between 1982 and 1987. Several patient characteristics were analyzed to determine whether they were associated with differences in survival, and the factors that emerged as significant were age, elevated serum lactate dehydrogenase (LDH), performance status, and number of extranodal sites of disease. The term may also include derived scores such as FLIPI, MIPI and age-adjusted IPI.

The expression "overall survival" or "OS" is well known to one of skill in the art and refers to the fate of the patient after diagnosis, despite the possibility that the cause of death in a patient is not directly due to the effects of the disease (cancer). In other words, it refers to the prognosis that the patient will not die because of DLBCL, preferably within at least 2 years, at least 3 years, at least 4 years, at least 5 years, at least 10 years, at least 15 years or at least 20 years. Most preferably, the patient will die for another cause than DLBCL.

The term "patient", as used herein, refers to a subject suffering from DLBCL, although the subject may not have been diagnosed yet. It may also refer to a subject not suffering from DLBCL who is to be subjected to the method of the invention nonetheless, e.g., to diagnose DLBCL or to serve as a control.

The term "performance status", as used herein, refers to the attempt to quantify cancer patients' general well-being and activities of daily life. This measure is used to determine whether they can receive chemotherapy, whether dose adjustment is necessary, and as a measure for the required intensity of palliative care. It is also used in oncological randomized controlled trials as a measure of quality of life. There are various scoring systems. The most generally used are the Karnofsky score and the Zubrod score, the latter being used in publications by the WHO. For children, the Lansky score is used.

The term "prognosis", as used herein, means the likelihood of recovery from a disease or the prediction of the probable development or outcome of a disease, including but not limited to predicting the length of overall survival (OS), 1 year survival (1YS), response to therapy (RT), disease-free survival, progression-free survival, and event-free survival. As will be understood by those skilled in the art, the prediction, although preferred to be, need not be correct for 100% of the subjects to be diagnosed or evaluated. The term, however, requires that a statistically significant portion of subjects can be identified as having an increased probability of having a given outcome. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90% at least 95%. The p-values are, preferably 0.05, 0.02, 0.01 or lower. Preferably, prognosis is characterized by predicting OS, survival at 1 year (1YS), and/or response to therapy (RT). The term "bad prognosis", as used herein, means an outcome which would be regarded negative for the patient and depends on the prognosis type; for example, a bad prognosis of 1 year survival (1YS) would mean that the patient will not survive for at least 1 year. The term "good prognosis", as used herein, means an outcome with would be regarded positive for the patient and depends on the prognosis type; for example, a good prognosis of 1 year survival (1YS) would mean that the patient will survive for at least 1 year. The expression "1 year survival (1YS)" refer to the prognosis that the patient will survive for at least 1 year.

The expression "progression-free survival" is well known to one of skill in the art and refers to the length of time during and after treatment in which a patient is living with a disease that does not get worse, and can be used in a clinical study or trial to help find out how well a treatment is working.

In the present invention, the expression "response to therapy" or "RT" relates to the response of the patient suffering from DLBCL to a therapy for treating said disease. Standard criteria (Miller, et al., Cancer, 1981; 47(1): 207-14) can be used herewith to evaluate the response to therapy include response, stabilization and progression. The term "response", as used herein, can be a complete response (or complete remission) which is the disappearance of all detectable malignant disease or a partial response which is defined as approximately >50% decrease in the sum of products of the largest perpendicular diameters of one or more lesions (tumor lesions), no new lesions and no progression of any lesion. Subjects achieving complete or partial response were considered "responders", and all other subjects were considered "non-responders". As will be understood by those skilled in the art, such an assessment is usually not intended to be correct for all (i.e. 100 percent) of the subjects to be identified. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley and Sons, New York 1983. Preferred confidence intervals are at least 90%, at least 95%, at least 97%, at least 98% or at least 99%. The p-values are, preferably, 0.1, 0.05, 0.01, 0.005, or 0.0001. More preferably, at least 60%, at least 70%, at least 80% or at least 90% of the subjects of a population can be properly identified by the method of the present invention. The term "stabilization", as used herein, is defined as a less than 50% (<50%) decrease or a less than 25% (<25%) increase in tumor size. The term "progression", as used herein, is defined as an increase in the size of tumor lesions by (more than 25%) >25% or appearance of new lesions.

The term "sample", as used herein, encompasses a variety of sample types obtained from a subject and useful in the procedure of the invention. Biological samples may include, but are not limited to, solid tissue samples, liquid tissue samples, biological fluids, aspirates, cells and cell fragments. Specific examples of biological samples include, but are not limited to, solid tissue samples obtained by surgical removal, a pathology specimen, an archived sample, or a biopsy specimen, tissue cultures or cells derived therefrom and the progeny thereof, and sections or smears prepared from any of these sources. Illustrative, non-limiting examples, include samples obtained from lymph nodes and other pathological specimens, wherein said pathological specimen are preferably characterized in that they contain DLBCL tumour material (e.g. cells, proteins, etc.), preferably the biomarkers of the invention derived from tumour tissue. One preferred such specimen is blood. Biological samples also include any material derived from the body of a mammal, including, but not limited to, blood, cerebrospinal fluid, serum, plasma, urine, nipple aspirate, fine needle aspirate, tissue lavage such as ductal lavage, saliva, sputum, ascites fluid, liver, kidney, breast, bone, bone marrow, testes, brain, ovary, skin, lung, prostate, thyroid, pancreas, cervix, stomach, intestine, colorectal, brain, bladder, colon, uterine, semen, lymph, vaginal pool, synovial fluid, spinal fluid, head and neck, nasopharynx tumors, amniotic fluid, breast milk, pulmonary sputum or surfactant, urine, fecal matter and other liquid samples of biologic origin, and may refer to either the cells or cell fragments suspended therein, or to the liquid medium and its solutes. All or a portion of the biological sample may have a level of biomarker expression characteristic of one or more disease state(s). Methods for obtaining the sample from the biopsy include gross apportioning of a mass, or microdissection, Tru-Cut biopsy or other art-known cell-separation methods. The cell or tissue sample is typically taken from the subject under study, namely, DLBCL patient previously diagnosed (or still not diagnosed) with DLBCL, or from a DLBCL patient undergoing treatment, etc. Methods of isolating cell and tissue samples are well known to those skilled in the art and include, but are not limited to, aspirations, tissue sections, needle biopsies, and the like. Frequently the sample will be a "clinical sample" which is a sample derived from a DLBCL patient, including sections of tissues such as frozen sections or paraffin sections taken for histological purposes. The sample can also be derived from supernatants (of cells) or the cells themselves taken from the DLBCL patients or from cell cultures, cells from tissue culture and fine needle aspirates.

The term "serum LDH", as used herein, refers to the level of LDH present in serum in a subject. Lactate dehydrogenase (LDH) is an enzyme (EC 1.1.1.27) present in a wide variety of organisms, including plants and animals. LDH catalyzes the interconversion of pyruvate and lactate with concomitant interconversion of NADH and NAD+. It converts pyruvate, the final product of glycolysis, to lactate when oxygen is absent or in short supply, and it performs the reverse reaction during the Cori cycle in the liver. At high concentrations of lactate, the enzyme exhibits feedback inhibition, and the rate of conversion of pyruvate to lactate is decreased. It also catalyzes the dehydrogenation of 2-hydroxybutyrate, but it is a much poorer substrate than lactate. Tissue breakdown elevates levels of LDH, and therefore a measure of it indicates, e.g., haemolysis. Other disorders indicated by elevated LDH include cancer, meningitis, encephalitis, acute pancreatitis, and HIV. It is used to follow-up cancer (especially lymphoma) patients, as cancer cells have a high rate of turnover with destroyed cells leading to an elevated LDH activity.

The expression "significantly associated with DLBCL prognosis", as used herein, refers to the unlikelihood of a result or event to be associated with DLBCL prognosis by chance. The amount of evidence required to accept that an event is unlikely to have arisen by chance is known as the significance level or critical p-value. Popular levels of significance are 10% (0.1), 5% (0.05), 1% (0.01), 0.5% (0.005), and 0.1% (0.001). Such results are informally referred to as 'statistically significant'. Choosing level of significance is an arbitrary task, but for many applications, a level of 5% is chosen, for no better reason than that it is conventional. In statistics, an association is any relationship between two measured quantities that renders them statistically dependent.

The term "subject", as used herein, includes any animal classified as a mammal and includes, but is not restricted to, domestic and farm animals, primates and humans, e.g., human beings, non-human primates, cows, horses, pigs, sheep, goats, dogs, cats or rodents. Preferably, the subject is a human being, male or female, of any age or race. In one embodiment, the patient has a tumour core of at least 10%, at least 20%, at least 30%, at least 40%, at least 50%, at least 60%, at least 70%, at least 80%, or at least 90%. The term "tumour core" has the meaning as generally known in the art. Preferably, it refers to the percentage of the tumour which is the core, the rest being the tumour periphery.

The term "tumor burden", as used herein, refers to the total mass of tumor tissue carried by an individual with cancer.

The term "VEGF", as used herein, refers to the human vascular endothelial growth factor A as shown in the UniProt database under accession number P15692 at date 4^{th} August 2011. In a preferred disclosure, the optimal cut-point value for VEGF is 0.18, although generally a range of 0.01-1.15 (0.0050 to 1.54, 95% confidence interval) is preferred, or alternatively, a range of 0.05 to 0.8 or 0.10 to 0.4, using the statistical method described in the examples (preferably Kaplan-Meier), p preferably ranging from 0.01 to 0.25, more preferably from 0.03 to 0.1 and most preferably being 0.05.

### Method for prognosis of a patient suffering from DLBCL

In a first aspect, the invention relates to an *in vitro* method for prognosis of a patient suffering from DLBCL, which comprises determining in a sample from said patient the level of BCL6 protein by quantitative immunofluorescence, wherein said patient is under therapy with CHOP-R, and wherein a high level of BCL6 expression in said sample is indicative of a bad prognosis and a low level of BCL6 expression in said sample is indicative of a good prognosis.

In a particular embodiment, the *in vitro* method for prognosis of a patient suffering from DLBCL further comprises determining in a sample from said patient the level of at least one biomarker selected from the group consisting of VEGF, CD68 and CD20.

In one aspect, the document discloses an *in vitro* method for prognosis of a patient suffering from diffuse large B-cell lymphoma (DLBCL), which comprises determining in a sample from said patient the level of at least one biomarker selected from the group consisting of VEGF, BCL6, CD68, CD20, and combinations thereof. Preferably,
- a high level of VEGF expression in said sample is indicative of a bad prognosis and a low level of VEGF expression in said sample is indicative of a good prognosis,
- a high level of BCL6 expression in said sample is indicative of a bad prognosis and a low level of BCL6 in said sample is indicative of a good prognosis,
- a high level of CD68 expression or CD68⁺ cells in said sample is indicative of a bad prognosis and a low level of CD68 expression or CD68⁺ cells in said sample is indicative of a good prognosis, and/or
- a high level of CD20 expression or CD20⁺ cells in said sample is indicative of a good prognosis and a low level of CD20 expression or CD20⁺ cells in said sample is indicative of a bad prognosis.

Thus, the document discloses an *in vitro* method for prognosis of a patient suffering from DLBCL, which comprises determining in a sample from said patient the level of at least one biomarker selected from the group consisting of VEGF, BCL6, CD68, CD20, and combinations thereof, wherein
- a high level of VEGF expression in said sample is indicative of a bad prognosis and a low level of VEGF expression in said sample is indicative of a good prognosis,
- a high level of BCL6 expression in said sample is indicative of a bad prognosis and a low level of BCL6 expression in said sample is indicative of a good prognosis,
- a high level of CD68 expression or CD68⁺ cells in said sample is indicative of a bad prognosis and a low level of CD68 expression or CD68⁺ cells in said sample is indicative of a good prognosis, and/or
- a high level of CD20 expression or CD20⁺ cells in said sample is indicative of a good prognosis and a low level of CD20 expression or CD20⁺ cells in said sample is indicative of a bad prognosis.

According to the invention, the term "determining the level of a biomarker" refers to determining the level of expression of a biomarker and/or the number of cells carrying a biomarker on its surface (i.e. a cell surface marker). Therein, the level of expression refers to the level of mRNA and/or the level of protein and/or the number of cells carrying a biomarker on its surface.

Methods for determining the expression can be based on determining the mRNA levels or protein levels in a sample as a whole, in cells of a sample and/or in the non-cellular fraction of a sample. Methods for determining mRNA levels are well known in the art and include, e.g., real-time PCR (rtPCR), northern blotting, nanostring and microarray technologies.

Virtually any conventional method can be used within the frame of the document to detect and quantify the levels of proteins. By way of a non-limiting illustration, the expression levels are determined by means of antibodies with the capacity for binding specifically to the protein to be determined (or to fragments thereof containing the antigenic determinants) and subsequent quantification of the resulting antigen-antibody complexes. The antibodies that are going to be used in this type of assay can be, for example polyclonal sera, hybridoma supernatants or monoclonal antibodies, antibody fragments, Fv, Fab, Fab' and F(ab')2, scFv, diabodies, triabodies, tetrabodies and humanized antibodies. At the same time, the antibodies may or may not be labeled. Illustrative, but non-exclusive, examples of markers that can be used include radioactive isotopes, enzymes, fluorophores, chemoluminescent reagents, enzyme cofactors or substrates, enzyme inhibitors, particles, dyes, etc. There is a wide variety of well known assays that can be used in the present document, using non-labeled antibodies (primary antibody) and labeled antibodies (secondary antibodies); these techniques include Western-blot or immunoblot, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, immunofluorescence, techniques based on the use of biochips or protein microarrays including specific antibodies or assays based on the colloidal precipitation in formats such as reagent strips. Other forms of detecting and quantifying the protein include affinity chromatography techniques, ligand-binding assays, etc.

In the invention, the determination of the levels of biomarkers is performed by quantitative immunofluorescence. Immunofluorescence (IF) is a technique used for light microscopy with a fluorescence microscope and is used primarily on biological samples. This technique uses the specificity of antibodies to their antigen to target fluorescent dyes to specific biomolecule targets within a cell, and therefore allows visualisation of the distribution of the target molecule through the sample. IF is a widely used example of immunostaining and is a specific example of immunohisto-chemistry (IHC) that makes use of fluorophores to visualise the location of the antibodies. IF can be used on tissue sections, cultured cell lines, or individual cells, and may be used to analyse the distribution of proteins, glycans, and small biological and non-biological molecules. IF can be used in combination with other, non-antibody methods of fluorescent staining, for example, use of DAPI to label DNA. Several microscope designs can be used for analysis of IF samples; the simplest is the epifluorescence microscope, and the confocal microscope is also widely used. Various super-resolution microscope designs that are capable of much higher resolution can also be used.

In one embodiment, said method further comprises determining and/or correlating at least one clinical parameter known to be indicative for DLBCL prognosis.

In a preferred disclosure, said biomarker is VEGF, said prognosis is optionally characterised by OS and said clinical parameter optionally is IPI and/or age.

In another preferred disclosure, said biomarker is VEGF, said prognosis is optionally characterised by 1YS and said clinical parameter optionally is IPI and/or age.

In another preferred disclosure, said biomarker is VEGF, said prognosis is optionally characterised by RT and said clinical parameter optionally is IPI and/or age.

In another preferred embodiment, said biomarker is BCL6, said prognosis is optionally characterised by OS and said clinical parameter optionally is IPI and/or age.

In another preferred embodiment, said biomarker is BCL6, said prognosis is optionally characterised by 1YS and said clinical parameter optionally is IPI and/or age.

In another preferred embodiment, said biomarker is BCL6, said prognosis is optionally characterised by RT and said clinical parameter optionally is IPI and/or age.

In another preferred disclosure, said biomarker is CD68, said prognosis is optionally characterised by OS and said clinical parameter optionally is IPI and/or age.

In another preferred disclosure, said biomarker is CD68, said prognosis is optionally characterised by 1YS and said clinical parameter optionally is IPI and/or age.

In another preferred disclosure, said biomarker is CD68, said prognosis is optionally characterised by RT and said clinical parameter optionally is IPI and/or age.

In another preferred disclosure, said biomarker is CD20, said prognosis is optionally characterised by OS and said clinical parameter optionally is IPI and/or age.

In another preferred disclosure, said biomarker is CD20, said prognosis is optionally characterised by 1YS and said clinical parameter optionally is IPI and/or age.

In another preferred disclosure, said biomarker is CD20, said prognosis is optionally characterised by RT and said clinical parameter optionally is IPI and/or age.

Since the determination of more than one biomarker can increase the probability of a correct prognosis or even enable the use of one biomarker which may not be sufficient on its own to provide a highly reliable prognosis, it is further envisaged that the method comprises the determination of the levels of BCL6 and one (or even more) biomarkers selected from the group consisting of VEGF, CD68, and CD20, and optionally one or more further biomarkers selected from that group.

Accordingly, in a preferred embodiment, said biomarkers are VEGF and BCL6 and optionally one or more of the biomarkers selected from the group consisting of CD68, and CD20, said prognosis is optionally characterised by OS and said clinical parameter optionally is IPI and/or age.

In another preferred embodiment, said biomarkers are VEGF and BCL6 and optionally one or more of the biomarkers selected from the group consisting of CD68, and CD20, said prognosis is optionally characterised by 1YS and said clinical parameter optionally is IPI and/or age.

In another preferred embodiment, said biomarkers are VEGF and BCL6 and optionally one or more of the biomarkers selected from the group consisting of CD68, and CD20, said prognosis is optionally characterised by RT and said clinical parameter optionally is IPI and/or age.

In another preferred disclosure, said biomarkers are VEGF and CD68 and optionally one or more of the biomarkers selected from the group consisting of BCL6, and CD20, said prognosis is optionally characterised by OS and said clinical parameter optionally is IPI and/or age.

In another preferred disclosure, said biomarkers are VEGF and CD68 and optionally one or more of the biomarkers selected from the group consisting of BCL6, and CD20, said prognosis is optionally characterised by 1YS and said clinical parameter optionally is IPI and/or age.

In another preferred disclosure, said biomarkers are VEGF and CD68 and optionally one or more of the biomarkers selected from the group consisting of BCL6, and CD20, said prognosis is optionally characterised by RT and said clinical parameter optionally is IPI and/or age.

In another preferred disclosure, said biomarkers are VEGF and CD20 and optionally one or more of the biomarkers selected from the group consisting of BCL6, and CD68, said prognosis is optionally characterised by OS and said clinical parameter optionally is IPI and/or age.

In another preferred disclosure, said biomarkers are VEGF and CD20 and optionally one or more of the biomarkers selected from the group consisting of BCL6, and CD68, said prognosis is optionally characterised by 1YS and said clinical parameter optionally is IPI and/or age.

In another preferred disclosure, said biomarkers are VEGF and CD20 and optionally one or more of the biomarkers selected from the group consisting of BCL6, and CD68, said prognosis is optionally characterised by RT and said clinical parameter optionally is IPI and/or age.

In another preferred embodiment, said biomarkers are BCL6 and CD68 and optionally one or more of the biomarkers selected from the group consisting of VEGF, and and CD20, said prognosis is optionally characterised by OS and said clinical parameter optionally is IPI and/or age.

In another preferred embodiment, said biomarkers are BCL6 and CD68 and optionally one or more of the biomarkers selected from the group consisting of VEGF, and CD20, said prognosis is optionally characterised by 1YS and said clinical parameter optionally is IPI and/or age.

In another preferred embodiment, said biomarkers are BCL6 and CD68 and optionally one or more of the biomarkers selected from the group consisting of VEGF, and CD20, said prognosis is optionally characterised by RT and said clinical parameter optionally is IPI and/or age.

In another preferred embodiment, said biomarkers are BCL6 and CD20 and optionally one or more of the biomarkers selected from the group consisting of VEGF, and CD68, said prognosis is optionally characterised by OS and said clinical parameter optionally is IPI and/or age.

In another preferred embodiment, said biomarkers are BCL6 and CD20 and optionally one or more of the biomarkers selected from the group consisting of VEGF, and CD68, said prognosis is optionally characterised by 1YS and said clinical parameter optionally is IPI and/or age.

In another preferred embodiment, said biomarkers are BCL6 and CD20 and optionally one or more of the biomarkers selected from the group consisting of VEGF, and CD68, said prognosis is optionally characterised by RT and said clinical parameter optionally is IPI and/or age.

In another preferred disclosure, said biomarkers are CD68 and CD20 and optionally one or more of the biomarkers selected from the group consisting of VEGF, and BCL6, said prognosis is optionally characterised by OS and said clinical parameter optionally is IPI and/or age.

In another preferred disclosure, said biomarkers are CD68 and CD20 and optionally one or more of the biomarkers selected from the group consisting of VEGF, and BCL6, said prognosis is optionally characterised by 1YS and said clinical parameter optionally is IPI and/or age.

In another preferred disclosure, said biomarkers are CD68 and CD20 and optionally one or more of the biomarkers selected from the group consisting of VEGF, and BCL6, said prognosis is optionally characterised by RT and said clinical parameter optionally is IPI and/or age.

In all above preferred embodiments and disclosures, in which said clinical parameter optionally is IPI and/or age, it may instead or additionally be selected from the group consisting of complete/partial response to therapy, rituximab treatment, gender, lack of recurrence in 1 year, nodal restricted disease, clinical stage (e.g. ECOG status), tumour burden, and serum LDH.

In the method of the first aspect, said patient is under a DLBCL therapy with cyclophosphamide (Cytoxan), doxorubicin (hydroxydaunorubicin), vincristine (Oncovin), and prednisone (CHOP), in combination with rituximab (CHOP-R), wherein said prognosis may be influenced by said therapy. The expression "may be influenced" in this respect means that the prognosis of said patient is affected by a response to the therapy the patient is subjected to or it is not affected by
a response to the therapy the patient is subjected, the latter either because there is no response to the therapy or because the response is not sufficient to change the prognosis.

Preferably, said DLBLC therapy is based on the administration of a drug, or combination of drugs, wherein said drug is selected from the group consisting of an alkylating agent, an anthracycline, an antimitotic vinca alkaloid, a corticosteroid, an anti-CD20 antibody and any combination thereof. More preferably, the alkylating agent is cyclophosphamide, the anthracycline is doxorubicin, the antimitotic vinca alkaloid is vincristine, the corticosteroid is prednisone, and the anti-CD20 antibody is rituximab.

An "alkylating agent", as used herein, is an agent that attaches an alkyl group (CₙH₂ₙ₊₁) to DNA; illustrative non-limitative examples of alkylating agents include cyclophosphamide, ifosfamide, thiotepa, busulfan, melphalan, chloroethylnitrosourea, mechlorethamin, chlorambucil and the like. In a particular embodiment, the alkylating agent is cyclophosphamide.

The term "anthracycline" or "anthracycline antibiotic", as used herein, refers to a class of drugs used in cancer chemotherapy derived from *Streptomyces* bacteria; said class of drugs has three mechanisms of action:
- inhibits DNA and RNA synthesis by intercalating between base pairs of the DNA/RNA strand, thus preventing the replication of rapidly-growing cancer cells;
- inhibits topoisomerase II enzyme, preventing the relaxing of supercoiled DNA and thus blocking DNA transcription and replication;
- creates iron-mediated free oxygen radicals that damage the DNA and cell membranes.

In a particular embodiment, the anthracycline is doxorubicine.

An "antimitotic vinca alkaloid" is a compounds that inhibits cell proliferation by binding to microtubules, which leads to a mitotic block and apoptosis. Illustrative, non-limiting, examples of antimitotic vinca alkaloids include vinblastine, vincristine, vindesine and navelbine. In a particular embodiment, the antimitotic vinca alkaloid is vincristine.

The term "corticosteroid" or corticoid, as used herein, refers to a class of steroid hormones that are produced in the adrenal cortex and their derivatives that are involved in a wide range of physiologic systems such as stress response, immune response and regulation of inflammation, carbohydrate metabolism, protein catabolism, blood electrolyte levels, and behavior. Illustrative, non-limiting, examples of corticosteroids include hydrocortisone, hydrocortisone acetate, cortisone acetate, tixocortol pivalate, prednisolone, methylprednisolone, prednisone, triamcinolone acetonide, triamcinolone alcohol, mometasone, amcinonide, budesonide, desonide, fluocinonide, fluocinolone acetonide, halcinonide, betamethasone, betamethasone sodium phosphate, dexamethasone, dexamethasone sodium phosphate, paramethasone, fludrocortisone and fluocortolone. In a particular embodiment, the corticosteroid is prednisone.

The term "anti-CD20 antibody", as used herein, refers to monoclonal antibodies or antigen-binding fragments thereof that specifically target CD20. Illustrative, non-limiting, examples of anti-CD20 antibodies include rituximab, ibritumomab tiuxetan, and tositumomab. In a particular embodiment, the anti-CD20 antibody is rituximab.

In a preferred embodiment, the alkylating agent is cyclophosphamide, the anthracycline is doxorubicin, the antimitotic vinca alkaloid is vincristine, the corticosteroid is prednisone, and the anti-CD20 antibody is rituximab. In a more preferred embodiment, treatment for DLBCL consists of cyclophosphamide (Cytoxan), doxorubicin (hydroxydaunorubicin), vincristine (Oncovin), and prednisone (CHOP), in combination with rituximab (CHOP-R).

In a further embodiment of the method of the first aspect, the level of said biomarker is compared to a cut-point value which is significantly associated with DLBCL prognosis with or without correlation to one or more clinical parameters and/or further biomarkers. As defined above, the expression "significantly associated with DLBCL prognosis", refers to the unlikelihood of a result or event to be associated with DLBCL prognosis by chance. The amount of evidence required to accept that an event is unlikely to have arisen by chance is known as the significance level or critical p-value. Popular levels of significance are 10% (0.1), 5% (0.05), 1% (0.01), 0.5% (0.005), and 0.1% (0.001). Such results are informally referred to as 'statistically significant'. Choosing level of significance is an arbitrary task, but for many applications, a level of 5% is chosen, for no better reason than that it is conventional. In statistics, an association is any relationship between two measured quantities that renders them statistically dependent.

It is envisaged that any biomarker or clinical parameter useful for DLBCL prognosis, preferably the ones mentioned herein, can be used together with the biomarkers BCL6. It is expected that the more biomarkers/clinical markers are used together, the more accurate the prediction will be and, also, the lower the demand on each of these biomarkers/clinical markers is (i.e. the cut-point value(s) can be less stringent), in particular on the biomarkers BCL6.

Preferably, said cut-point value has been predetermined or is determined from a cohort of patients with known DLBCL outcome. More preferably, said cut-point value has been predetermined or is determined such that the probability of an incorrect prognosis is less than 5%, even more preferably less than 4%, less than 3%, less than 2%, less than 1% or less than 0.5%. The expression "known DLBCL outcome", as used herein, refers to the parameters used to evaluate the development or outcome of a disease that have been recorded for a patient. Examples of parameters include, but are not limited to, the length of OS, OS at 1 year, disease-free survival, progression-free survival, event-free survival, age, gender, ISI and, in fact all clinical parameters or prognosis types mentioned herein.

As laid out above, the probability of an incorrect prognosis depends not only on one cut-point value if more than one biomarker/clinical marker is used together. Generally, it is envisaged that the probability of an incorrect prognosis is less than 5% taking into account all biomarkers/clinical markers and their respective cut-point values. Thus, if more than one biomarker/clinical marker is used together, each cut-point value can be predetermined or determined such that the probability of an incorrect prognosis is more than 5% for each individual cut-point, e.g. less than 40%, less than 30%, less than 20%, less than 10%, but also less than 5%, less than 4%, less than 3%, less than 2%, less than 1% or less than 0.5%.

Accordingly, the cut-point value is a statistical value, which represents a value correlated to the biomarker level at which the probability of an incorrect prognosis has a certain value represented by the p-value, e.g. a p-value of 0.5 for a probability of an incorrect prognosis of less than 5%. Said cut-point value depends on the statistical method used and, therefore, the scope of the invention would be unduly limited by specifying cut-point values. Also, the cut-point value depends on the type of prognosis, e.g. it may be different for OS or 1YS. Thus, all such values provided herein are merely exemplary for the statistical method and the purpose they were acquired with.

Ultimately, the cut-point value represents a biomarker level which is high or low enough to provide a prognosis with a particular probability of being incorrect. Of course, this biomarker level depends also on the type of prognosis and on further variables such as the cohort of patients used to derive the cut-point value from, and therefore, specific levels indicated herein are also to be considered exemplary. Generally, it is envisaged that the cut-point value may mean a biomarker (expression or cell count) level increased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more (i.e. "high level" in the sense of the present invention). Similarly, it may mean a level decreased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, i.e., absent (i.e. "low level" in the sense of the present invention).

In other words, the term "cut-point", as used herein, refers to a mathematically defined biomarker level which optimizes sensitivity and specificity for stratifying patients as high or low risk for a particular outcome, i.e. OS. The cut-point is derived after the identification of a biomarker threshold which assigns a positive or negative signal value for any biomarker under study. The biomarker threshold can be established by a person of average skill in the art, using an analysis software algorithm to identify a cell compartment associated positive signal pixel area from background, autofluorescence and non-specific binding of either the antibody or the fluorochrome.

Statistical methods suitable for carrying out the method of the first aspect are known by the skilled person and can be performed without undue burden. Such tests include, but are not limited to cox regression analysis, Kaplan-Meier survival curves, area under the curve (AUC), concordance index and Hazard ratio.

### DLBCL patient selection for therapy

In a second aspect, the present document discloses a method for selecting a patient suffering from DLBCL for individual therapy, comprising the method of the first aspect potentially relating to any one of above-described embodiments, wherein said patient is under a DLBCL therapy or not, and wherein a good prognosis selects said patient for said individual therapy and/or a bad prognosis rejects said patient for said individual therapy.

The term "therapy", as used herein, refers to a therapeutic treatment, as well as a prophylactic or prevention method, wherein the goal is to prevent or reduce an unwanted physiological change or disease, such as cancer. Beneficial or desired clinical results include, but not limiting, release of symptoms, reduction of the length of the disease, stabilized pathological state (specifically not deteriorated), retard in the disease's progression, improve of the pathological state and remission (both partial and total), both detectable and not detectable. The terms "treat" and "treatment" are synonyms of the term "therapy" and can be used without distinction along the present description. "Treatment" can mean also prolong survival, compared to the expected survival if the treatment is not applied. In the sense of the present document, the term "therapy" means a therapy meaning to be beneficial, it does however not mean that it is necessarily beneficial for an individual. As such, it is a treatment thought to be beneficial for at least some DLBCL patients. The term "individual therapy", as used herein, refers to the therapy for which an individual patient is, but necessarily other or even all patients are predicted to have a good prognosis.

The term "selecting", as used herein, refers to the action of choosing a patient suffering from DLBCL for an individual therapy, and it is based on the evaluation of the prognosis of said patient. It does, however, not refer to merely trying a therapy on a patient to find out, for example, using the method of the invention, whether the therapy is suitable for that particular patient.

Thus, the method of a second aspect allows determining whether a particular DLBCL therapy is useful for treating an individual patient. This is particularly important since not all known DLBCL therapies are suitable for treating all patients.

In one disclosure of the method of the second aspect, said method is repeated with said patient under (i) a DLBCL therapy if not yet under a DLBCL therapy or (ii) a
different DLBCL if already under a DLBCL therapy, preferably until said patient is selected for said individual therapy.

Accordingly, the method of the second aspect can be carried out until a DLBCL therapy is found which is suitable for treating a particular patient. Therein, the patient can be subjected to several treatments, in succession or in combination, to find out which therapy is useful for him.

### Kits

In a third aspect, the present document discloses a kit comprising reagents for analyzing a sample from a subject for determining the levels of one or more biomarkers selected from the group consisting of VEGF, BCL6, CD68, and CD20 according to the methods of the first and/or the second aspect potentially relating to any one of above-described embodiments and disclosures.

As used herein, the term "kit" is used in reference to a combination of articles that facilitate the methods of the present invention. These kits provide the materials necessary for carrying out the application described herein.

The kit may comprise, in addition, a packaging which allows maintaining the reagents within determined limits. Suitable materials for preparing such packings include glass, plastic (polyethylene, polypropylene, polycarbonate and the like), bottles, vials, paper, sachets and the like. The kit of the document can additionally contain instructions for using the components contained therein. Said instructions can be found in the form of printed material or in the form of an electronic support which can store instructions such that they can be read by a subject, such as electronic storage media (magnetic disks, tapes and the like), optical media (CD-ROM, DVD) and the like. The media can additionally or alternatively contain Internet websites providing said instructions.

The use of said kit for prognosis of a patient suffering from diffuse large B-cell lymphoma (DLBCL) or for selecting a patient suffering from DLBCL for individual therapy, constitutes further disclosures of this document.

The invention is described by way of the following example which is to be construed as merely illustrative and not limitative of the scope of the invention.

### EXAMPLE 1

### Identification of markers associated to prognosis and response to therapy in patients with DLBCL

### I. MATERIALS AND METHODS

### Patients/Samples

Clinical data and un-stained Tissue Microarray (TMA) slides for 149 patients with diffuse large-B-cell lymphoma (DLBCL) from the Hematopathology Unit of the Hospital Clinic, University of Barcelona (Dr. Elias Campo) were received by the inventors. All the patients had complete clinical data and available tumor for incorporation into 8 TMA blocks.

This study was approved by the Hospital Clinic ethics institutional review board. The majority of patients had complete clinical record files which included: age, gender, location, International Prognostics Index (IPI) stage, treatment, response and outcome.

It was assumed that all patients were treated with either rituximab and/or cyclophosphamide, doxorubicin, vincristine, and prednisone (CHOP) or CHOP-like therapies although the clinical data files only capture rituximab treatment.

### Quantitative Multiplex Immunofluorescence (QMIF)

Immunofluorescent assays were performed and analyzed by a certified pathologist at a clinical commercial laboratory (Althia, Barcelona). Briefly, individual TMA slides were stained with Hematoxylin and Eosin (H&E) and evaluated by two pathologists for overall tumor content and quality. Upon completion of initial quality assessment 3 multiplex immunofluorescent assays were performed which included the following antibodies grouped according to prioritization and multiplex format (Tables 1, 2, and 3): MUM1, Ki67, CD20, CD34, BCL2, BCL6, CD68, VEGF, HLA-DR, CMYC and NfKB. All samples were processed utilizing previously developed immunofluorescent assays (Cordon-Cardo et al., J Clin Invest. 2007;117:1876-83) and evaluated with the CRI Nuance imaging software. Individual fluorescent thresholds and co-localization strategies were employed to develop specific features for assessment with respect to outcome. Some of these features included the mean, median, max levels of CD20, Ki67 and CD34 based on area and intensity expression values, and the evaluation of selected cell populations in conjunction with other markers, i.e. amount, presence, intensity, and area of CD20 expressing cells which were also Ki67 positive.

**Table 1**

| **Lymphoma multiplex I** | | | | | |
|---|---|---|---|---|---|
| **Antibody** | **Vendor** | **Catalog #** | **Dilution** | **Isotype** | **Labels** |
| MUM-1 | DAKO | | 1:25 | M IgG1 | 2X M 488 |
| Ki67 | DAKO | | 1:100 | M IgG1 | 2X M 555 |
| CD20 | DAKO | | 1:300 | M IgG2A | 2X M2A 568 |
| CD34 | DAKO | | 1:50 | M IgG1 | 2X M 594 |

**Table 2**

| **Lymphoma Multiplex II** | | | | | |
|---|---|---|---|---|---|
| **Antibody** | **Vendor** | **Catalog #** | **Dilution** | **Isotype** | **Labels** |
| BCL2 | DAKO | M0997 | 1:20 | M IgG1 | 2X M 488 |
| BCL6 | DAKO | IS625 | RTU | M IgG1 | 2X M 568 |
| CD68 | DAKO | M0814 | 1:100 | M IgG1 | ¼ XM 594 |
| VEGF | ABCAM | AB1316 | 1:400 | M IgG1 | 2XM555 |

**Table 3**

| **Lymphoma Multiplex III** | | | | | |
|---|---|---|---|---|---|
| **Antibody** | **Vendor** | **Catalog #** | **Dilution** | **Isotype** | **Labels** |
| C-myc | ABCAM | Ab39688 | 1:25 | R IgG | 2X R 488 |
| HLA-DR | DAKO | M0746 | 1:20 | M IgG1 | 2X M 555 |
| NFkB | SANTACRUZ | SC-372 | 1:50 | R IgG1 | 2X R 594 |

A series of 3-5 antibodies per multiplex (Tables 1-3) were combined with DAPI (4',6-diamidino-2-phenylindole) to produce a series of multiplex assays. Briefly, the analysis included utilizing the appropriate antibodies in the order they are listed in the previous tables according to the specified dilutions and Alexa fluorescent dyes. The antibody mixture was applied to the tissue sample, and the antibodies were allowed to bind in a humid chamber at room temperature for one hour. Incubation was followed by two rinses of six minutes each in PBT (PBS + Tween), one rinse of six minutes in PBS (phosphate buffered solution), and one rinse of three minutes in PBS.

For the individual labeling steps, a cocktail of Zenon Alexa Fluor 488 anti-Rabbit, IgG Fab fragment Zenon Alexa Fluor 555 anti-mouse IgG1 Fab fragment, and Zenon Alexa Fluor 568 anti-mouse IgG1 Fab fragment, and Zenon Alexa Fluor 594 anti-mouse IgG2a Fab fragment was made in 1% Blocking Reagent at twice the concentrations recommended by the manufacturer (1:50 dilution for each Fab fragment). Approximately 100 µl of this labeling cocktail was applied to the tissue samples, and the tissue samples were incubated in a humid chamber at room temperature for 30 minutes. The labeling reaction was followed by two rinses of six minutes each in PBT, one rinse of six minutes in PBS, and one rinse of three minutes in PBS.

Images were acquired with a CRI Nuance spectral imaging system (CRI, Inc., 420-720 nm model) mounted on a Nikon 90i microscope equipped with a mercury light source (Nikon). DAPI nuclear counterstain was recorded at 480 nm wavelength using a bandpass DAPI filter (Chroma). Alexa 488 was captured between 520 and 560 nm in 10 nm intervals using an FITC filter (Chroma). Alexa 555, 568 and 594 were recorded between 570 and 670 nm in 10 nm intervals using a custom-made longpass filter (Chroma). Spectra of the pure dyes were recorded prior to the experiment by diluting each Alexa dye separately in SlowFade Antifade (Molecular Probes). The diluted dye was then spread out on a glass slide, covered with a coverslip and scanned with the same range and interval as the respective dye in the tissue experiment. Representative regions of background fluorescence were allocated in order to complete the spectral libraries for the spectral unmixing process. Images were unmixed using the Nuance software Version 1.4.2. The resulting images were saved as quantitative grayscale tiff images and submitted for analysis.

The results of the immunoflorescent assays were evaluated for association with either response to treatment and/or survival using Kaplan-Meier survival function curves, hazard ratio and when appropriate the concordance index (CoI).

### Multivariate models

A variety of multivariate clinical and immunofluorescence (IF) models were constructed to evaluate associative predictors and potentially overlapping pathways that may be synergistic with respect to outcome. The models were based on 58 patients with complete data sets and incorporated protein IF profiles that were univariately significant or trended towards association with OS. The markers that were selected included BCL6, CD68, CD20, VEGF (optimal cut-point) along with the complete clinical variables including age, gender, stage, and IPI score. The individual feature selection results were based on Bayesian Information Criterion approach.

### II. RESULTS

### Patient profile

The cohort consisted of 149 patients, median age 61 years, 43% female, 57% male, and the IPI breakdown was as follows: stage I (30%), II (23%), III (25%) and stage IV (22%). Clinical factors significantly associated with improved overall survival (OS) included: complete/partial response to therapy, rituximab treatment, female gender, age<60 years, lack of recurrence in year 1, nodal restricted disease, lower stage, good ECOG status, bulky disease, and low IPI score (1/II).

### Quantitative multiplex immunofluorescence (QMIF)

Inventors then compared individual markers with specific clinical variables. Only CD20 by QMIF was associated with stage (p = 0.024) and LDH levels (p = 0.016); and CD68 was associated with age (p = 0.026). No markers were associated with extranodal distribution or ECOG status. Of significance, when the complete biomarker set (MUM1, Ki67, CD20, CD34, BCL2, BCL6, CD68, VEGF, HLA-DR, CMYC and NfKB) was evaluated with response (e.g. complete/partial vs. none), n = 67 patients, only the percentage of intratumoral CD68+ cells was significant relative to tumor area, i.e. the greater the number of CD68+ cells the worse the outcome (p = 0.019), i.e., increasing tumoral-CD68+ cells were associated with a poor response to treatment (complete/partial vs. none; p=0.019). Of note, in the present study, CD68+ macrophages were not associated with OS (p = 0.9) nor survival at 1 year (p = 0.88) (Fig. 1A and 1B).

Although 87% of the patients had received rituximab, CD20+ expression was not associated with OS (p = 0.32) nor survival at 1 year (p = 0.27) (Fig. 1C and 1D). This was not surprising as approximately 30% of patients who were CD20+ by flow cytometry were resistant or appeared not to have a favorable response to rituximab for various reasons. When CD68+ macrophages were combined with CD20+ cells using their respective cut-points, it was found that patients with combined increasing CD20+ cells and low numbers of CD68+ macrophages appeared to have improved OS (p = 0.13), although not statistically significant (Fig. 1D), but not improved survival at 1 year (p = 0.28) (Fig. 1E). This was not observed when these markers were evaluated independently.

The only marker, measured on a continuous scale, which was univariately predictive for OS was VEGF [HR (Hazard Risk) 1.23, 95% CI (Confidence Interval) [0.98-1.54], p = 0.010, CoI (Concordance Index) 0.34]. Of importance, when the optimal VEGF cut-point (0.18) was applied the association of VEGF with OS exhibited improved significance (Figure 2A) (comparable to AUC).

The only additional marker measured as a continuous variable which was marginally associated with survival at only 1 year was BCL6 [HR 0.55, 95% CI [0.20-1.56], p = 0.087, CoI 0.43]. However, a BLC6 cut-point of 3.84 was found to be significantly associated with OS (Figure 3A).

### Multivariate models

Two models were generated with good results for predicting OS:
- Model 1: Age (HR 1.04, p = 0.065), IPI (HR 1.72, p = 0.035), and VEGF (2.63, p = 0.063) (Best Model).
- Model 2: Age (HR 1.04, p = 0.64), IPI (HR 1.89, p = 0.017), BCL6 (HR 1.08, p = 0.11), and VEGF (HR 3.04, p = 0.038).

The best performing model suggests that age, IPI and VEGF are potentially the most important variables useful for predicting OS. The CoI for Model 1 was 0.78. Of interest, when the inventors substituted the QMIF BCL6 with the data derived from immunohistochemistry (IHC) BCL6 provided with the clinical data, the IHC BCL6 was not selected thus providing additional support for the use of quantitative methods to assess biomarkers in human tissue specimens.

### Conclusions

In view of the results obtained, it can be concluded that:
a) several interesting profiles have been identified including the association of CD68+ macrophages for predicting response to therapy and the expression levels of VEGF and/or BCL6 as important predictors of OS in patients with DLBCL treated with CHOP-rituximab and followed for a median of 4 years; and
b) the combination of high levels of CD20 expressing tumor cells (CD20+ cells) and low CD68+ macrophages may be important for identifying a phenotype of good response to therapy.

## Claims

1. An *in vitro* method for prognosis of a patient suffering from diffuse large B-cell lymphoma (DLBCL), which comprises determining in a sample from said patient the level of BCL6 protein by quantitative immunofluorescence, wherein said patient is under therapy with CHOP-R, and wherein a high level of BCL6 expression in said sample is indicative of a bad prognosis and a low level of BCL6 expression in said sample is indicative of a good prognosis.

2. The method of claim 1, further comprising determining in a sample from said patient the level of at least one biomarker selected from the group consisting of VEGF, CD68 and CD20.

3. Method according to any one of claims 1 or 2, wherein said method further comprises determining and/or correlating at least one clinical parameter known to be indicative for DLBCL prognosis.

4. Method according to claim 3, wherein said clinical parameter is selected from the group consisting of international prognostic index (IPI), age, complete/partial response to therapy, rituximab treatment, gender, lack of recurrence in 1 year, nodal restricted disease, clinical stage, tumour burden, and serum lactate dehydrogenase (LDH).

5. Method according to claim 4, wherein said clinical parameter is IPI and/or age.

6. Method according to any one of claims 1 to 5, wherein said prognosis is **characterised by** predicting overall survival (OS), predicting survival at 1 year, and/or response to therapy.

7. Method according to any one of claims 1 to 6, wherein the level of said biomarker is compared to a cut-point value which is significantly associated with DLBCL prognosis with or without correlation to one or more clinical parameters and/or further biomarkers.

8. Method according to claim 7, wherein said cut-point value has been predetermined or is determined from a cohort of patients with known DLBCL outcome.

9. Method according to claim 8, wherein said cut-point value has been predetermined or is determined such that the probability of an incorrect prognosis is less than 5%.

## Patentansprüche

1. *In vitro* Verfahren zur Prognose eines Patienten, der unter diffusem großzelligen B-Zell-Lymphom (DLBCL) leidet, welches das Bestimmen des Spiegels von BCL6-Protein in einer Probe des Patienten mittels quantitativer Immunofluoreszenz umfasst, wobei der Patient unter Therapie mit CHOP-R ist und wobei ein hohes Expressionsniveau von BCL6 in der Probe indikativ für eine schlechte Prognose ist und ein niedriges Expressionsniveau von BCL6 in der Probe indikativ für eine gute Prognose ist.

2. Verfahren nach Anspruch 1, das weiterhin das Bestimmen von zumindest einem Biomarker, ausgewählt aus der Gruppe, die aus VEGF, CD68 und CD20 besteht, in einer Probe des Patienten umfasst.

3. Verfahren nach einem der Ansprüche 1 oder 2, wobei das Verfahren weiterhin das Bestimmen und/oder Korrelieren zumindest eines klinischen Parameters umfasst, von dem bekannt ist, das er indikativ für die DLBCL-Prognose ist.

4. Verfahren nach Anspruch 3, wobei der klinische Parameter ausgewählt ist aus der Gruppe bestehend aus Internationalem prognostischen Index (IPI), Alter, komplettes/teilweises Ansprechen auf Therapie, Rituximab-Behandlung, Geschlecht, keinem erneuten Auftreten in 1 Jahr, auf Knoten eingeschränkte Krankheit, klinisches Stadium, Tumorlast und Serum Lactatdehydrogenase (LDH).

5. Verfahren nach Anspruch 4, wobei der klinische Parameter IPI und/oder Alter ist.

6. Verfahren nach einem der Ansprüche 1 bis 5, wobei die Prognose durch das Vorhersagen des Gesamtüberlebens (OS), Vorhersagen des Überlebens nach 1 Jahr und/oder Ansprechen auf Therapie gekennzeichnet ist.

7. Verfahren nach einem der Ansprüche 1 bis 6, wobei der Spiegel des Biomarkers mit einem Grenzwert verglichen wird, welcher signifikant mit der DLBCL-Prognose assoziiert ist, mit oder ohne Korrelation zu einem oder mehreren klinischen Parametern und/oder weiteren Biomarkern.

8. Verfahren nach Anspruch 7, wobei der Grenzwert vorher festgelegt wurde oder von einer Kohorte von Patienten mit bekanntem DLBCL-Ausgang bestimmt wird.

9. Verfahren nach Anspruch 8, wobei der Grenzwert vorher festgelegt wurde oder derart festgelegt wird, dass die Wahrscheinlichkeit einer falschen Prognose kleiner als 5% ist.

## Revendications

1. Un procédé *in vitro* pour le pronostic d'un patient atteint d'un lymphome diffus à grandes cellules B (LDGCB), qui comprend la détermination dans un prélèvement dudit patient du niveau de la protéine BCL6 par immunofluorescence quantitative, dans lequel ledit patient est sous traitement CHOP-R, et dans lequel un niveau élevé d'expression de la BCL6 dans ledit prélèvement est indicatif d'un mauvais pronostic et un niveau faible d'expression de la BCL6 dans ledit prélèvement est indicatif d'un bon pronostic.

2. Le procédé selon la revendication 1, comprenant en outre la détermination dans un prélèvement dudit patient du niveau d'au moins un biomarqueur sélectionné parmi le groupe constitué de VEGF, CD68 et CD20.

3. Procédé selon l'une quelconque des revendications 1 ou 2, dans lequel ledit procédé comprend en outre la détermination et/ou la corrélation d'au moins un paramètre clinique connu pour être indicatif du pronostic LDGCB.

4. Procédé selon la revendication 3, dans lequel ledit paramètre clinique est sélectionné parmi le groupe constitué de l'indice pronostic international (IPI), l'âge, la réponse complète/partielle à la thérapie, le traitement par rituximab, le sexe, l'absence de récidive en un an, l'atteinte ganglionnaire isolée, le stade clinique, la masse tumorale, et la lacticodéshydrogénase (LDH) sérique.

5. Procédé selon la revendication 4, dans lequel ledit paramètre clinique est l'IPI et/ou l'âge.

6. Procédé selon l'une quelconque des revendications 1 à 5, dans lequel ledit pronostic est **caractérisé par** la prédiction de la survie globale (SG), la prédiction de la survie à 1 an, et/ou la réponse à la thérapie.

7. Procédé selon l'une quelconque des revendications 1 à 6, dans lequel le niveau dudit biomarqueur est comparé à une valeur seuil qui est associée de façon significative avec un pronostic LDGCB avec ou sans corrélation avec un ou plusieurs paramètres cliniques et/ou d'autres biomarqueurs.

8. Procédé selon la revendication 7, dans lequel ladite valeur seuil a été prédéterminée ou est déterminée à partir d'une cohorte de patients dont l'évolution du LDGCB est connue.

9. Procédé selon la revendication 8, dans lequel ladite valeur seuil a été prédéterminée ou est déterminée de telle sorte que la probabilité d'un pronostic erroné est inférieure à 5 %.
